# EUROPEAN PATENT APPLICATION

(11) **EP 2 286 800 A1**
(43) Date of publication of application: **23.02.2011**
(21) Application number: 10178627.5
(22) Date of filing: 11.04.2006
(51) Int. Cl.: A61K 9/20, A61K 31/47, A61P 31/04, A61K 9/48

(54) **Pharmaceutical compositions having improved dissolution profiles for poorly soluble drugs**

(30) Priority: 11.04.2005 US 670207 P
(62) Divisional of application: 06740898.9
(71) Applicant: Abbott Laboratories, Abbott Park, Illinois 60064-6008 (US)
(72) Inventor: Wu, Huailiang, Long Grove, IL 60047 (US); Zhang, Geoff, Libertyville, IL 60048 (US)
(74) Representative: Modiano, Micaela Nadia

(57) **Abstract**

Pharmaceutical compositions having improved dissolution profiles for drugs therein is disclosed.

## Description

### FIELD OF THE INVENTION

This invention pertains to pharmaceutical compositions having improved dissolution profiles for drugs therein.

### BACKGROUND OF THE INVENTION

Absorption of poorly soluble drugs is a major obstacle in pharmaceutical development. For ionizable compounds, salt formation is often used to improve solubility. This approach, however, may not work for compounds that precipitatate at lower pH, such as gastric pH. There is therefore an existing need in the pharmaceutical development arts for compositions which provide improved dissolution profiles of drugs.

### BRIEF DESCRIPTION OF THE FIGURES

FIG. 1 shows dissolution profiles of formulations containing various amounts of crystallization inhibitor for EXAMPLE 1.
FIG. 2 shows dissolution profiles of formulations containing a pH modifier and a crystallization inhibitor for EXAMPLE 1.
FIG. 3 shows dissolution profiles of formulations containing a pH modifier and a crystallization inhibitor for 5,5-diphenylhydantoin, sodium salt.
FIG. 4 shows dissolution profiles of formulations containing a pH modifier and a crystallization inhibitor for potassium mefenamate.

### SUMMARY OF THE INVENTION

Accordingly, one embodiment of this invention comprises compositions comprising an ionizable drug or a salt thereof, a crystallization inhibitor and a pH modifier, said composition providing a measurable improvement in dissolution rate of the ionizable drug.

Another embodiment of this invention comprises compositions comprising an ionizable drug or a salt thereof, a crystallization inhibitor and a pH modifier, said composition providing a reduced variability in dissolution.

Another embodiment comprises compositions comprising a salt of an ionizable drug, a crystallization inhibitor and a pH modifier, said composition providing a measurable improvement in dissolution rate of the ionizable drug.

Still another embodiment comprises compositions comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof, a crystallization inhibitor and a pH modifier, said composition providing a measurable improvement in dissolution rate of the ionizable drug.

Still another embodiment comprises compositions comprising the megluamine salt of 1-(6-amino-3,5-difluoropyridin-2-yl)-8-chloro-6-fluoro-7-(3-hydroxyazetidin-1-yl)-4-oxo-1,4-dihych-oquinoline-3-carboxylic acid, a crystallization inhibitor and a pH modifier, said composition providing a measurable improvement in dissolution rate of the ionizable drug.

Another embodiment comprises a method for treating disease in a patient comprising administering thereto a composition comprising an ionizable drug or a salt thereof, a crystallization inhibitor and a pH modifier, said composition providing a measurable improvement in dissolution rate of the ionizable drug.

Another embodiment comprises a method for treating disease in a patient comprising administering thereto a composition comprising an ionizable drug or a salt thereof, a crystallization inhibitor and a pH modifier, said composition providing a reduced variability in dissolution.

Another embodiment comprises a method for treating disease in a patient comprising administering thereto a composition comprising an ionizable drug, a crystallization inhibitor and a pH modifier, said composition providing a measurable improvement in dissolution rate of the ionizable drug.

Another embodiment comprises a method for treating disease in a patient comprising administering thereto a composition comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof, a crystallization inhibitor and a pH modifier, said composition providing a measurable improvement in dissolution rate of the ionizable drug.

Another embodiment comprises a method for treating disease in a patient comprising administering thereto a composition comprising the megluamine salt of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid, a crystallization inhibitor and a pH modifier, said composition providing a measurable improvement in dissolution rate of the ionizable drug.

### DETAILED DESCRIPTION OF THE INVENTION

The term "crystallization inhibitor," as used herein, means an agent that facilitates prevention of precipitation of a drug in a composition comprising the agent, a pH modifier and the drug. Examples of crystallization inhibitors include, but are not limited to hydroxypropylmethylcelluose, polyvinypyrrolidone, hydroxypropylcelluose and the like.

The term "ionizable drug," as used herein, means a compound having an acidic or basic moiety that is useful for treating a disease state or an adverse physiological event.

The term "measurable improvement in dissolution rate," as used herein, means at least a 5% increase in dissolution rate at a particular temperature and pH.

The term "pH modifier," as used herein, means a compound that is capable of changing the pH of a solution. Examples of pH modifiers include, but are not limited to NaOH, LiOH, KOH, Na₂CO₂, NaHCO₃, K₂CO₃, KHCO₃, NaH₂PO₄, Na₂HPO₄, Na₃PO₄, KH₂PO₄, K₂HPO₄, K₃PO₄, megluamine, Ca(OH)₂, Mg(OH)₂, Zn(OH)₂, Al(OH)₃, pyridoxine, triethanolamine, ammonium hydroxide, cytosine, diethylamine, meglumine, ornithine, glycine, lysine, arginine, valine, proline, aspartic acid, alanine, asparagine, isoleucine, leucine, methionine, threonine, choline hydroxide, procaine, diethylethanolamine, glucosamine, guanine, nicotinamide, piperazine, guanidine, olamine, piperidine, triethylamine, tromethamine, benzathine, benzathine, adenine, mixtures thereof and the like.

The term "pharmaceutical compositions," as used herein, means a combination of substances, at least one of which is a drug, or a therapeutically acceptable salt thereof The term "q.s.," as used herein, means as much as suffices.

Compounds having formula (I) may exist as acid addition salts, basic addition salts or zwitterions. Salts of compounds having formula (I) are prepared during their isolation or following their purification. Acid addition salts are those derived from the reaction of a compound having formula (I) with acid. Accordingly, salts including the acetate, adipate, alginate, bicarbonate, citrate, aspartate, benzoate, benzenesulfonate, bisulfate, butyrate, camphorate, camphorsufonate, digluconate, formate, fumarate, glycerophosphate, glutamate, hemisulfate, heptanoate, hexanoate, hydrochloride, hydrobromide, hydroiodide, lactobionate, lactate, maleate, mesitylenesulfonate, methanesulfonate, megluamine, naphthylenesulfonate, nicotinate, oxalate, pamoate, pectinate, persulfate, phosphate, picrate, propionate, succinate, tartrate, thiocyanate, trichloroacetic, trifluoroacetic, paratoluenesulfonate and undecanoate salts of the compounds having formula (I) are meant to be embraced by this invention. Basic addition salts of compounds are those derived from the reaction of the compounds having formula (I) with the bicarbonate, carbonate, hydroxide or phosphate of cations such as lithium, sodium, potassium, calcium and magnesium.

Compounds having formula (I) may be administered, for example, bucally, ophthalmically, orally, osmotically, parenterally (intramuscularly, intraperintoneally intrasternally, intravenously, subcutaneously), rectally, topically, transdermally, vaginally and intraarterially as well as by intraarticular injection, infusion, and placement in the body, such as, for example, the vasculature.

Therapeutically effective amounts of the drugs of this invention depend on recipient of treatment, disease treated and severity thereof, composition comprising it, time of administration, route of administration, duration of treatment, potency, rate of clearance and whether or not another drug is co-administered. The amount of a compound having formula (I) used to make a composition to be administered daily to a patient in a single dose or in divided doses is from about 0.03 to about 200 mg/kg body weight. Single dose compositions contain these amounts or a combination of submultiples thereof.

Compounds having formula (I) may be administered with or without an excipient. Excipients include, but are not limited to, encapsulators and additives such as absorption accelerators, antioxidants, binders, buffers, coating agents, coloring agents, diluents, disintegrating agents, emulsifiers, extenders, fillers, flavoring agents, humectants, lubricants, perfumes, preservatives, propellants, releasing agents, sterilizing agents, sweeteners, solubilizers, wetting agents, mixtures thereof and the like.

Excipients for preparation of compositions comprising a compound having formula (I) to be administered orally include, but are not limited to, agar, alginic acid, aluminum hydroxide, benzyl alcohol, benzyl benzoate, 1,3-butylene glycol, carbomers, castor oil, cellulose, cellulose acetate, cocoa butter, corn starch, corn oil, cottonseed oil, crosspovidone, diglycerides, ethanol, ethyl cellulose, ethyl laureate, ethyl oleate, fatty acid esters, gelatin, germ oil, glucose, glycerol, groundnut oil, isopropanol, isotonic saline, lactose, magnesium hydroxide, magnesium stearate, malt, mannitol, monoglycerides, olive oil, peanut oil, potassium phosphate salts, potato starch, povidone, propylene glycol, Ringer's solution, safflower oil, sesame oil, sodium carboxymethyl cellulose, sodium phosphate salts, sodium lauryl sulfate, sodium sorbitol, soybean oil, stearic acids, stearyl fumarate, sucrose, surfactants, talc, tragacanth, tetrahydrofurfuryl alcohol, triglycerides, water, mixtures thereof and the like, Excipients for preparation of compositions comprising a compound having formula (I) to be administered ophthalmically or orally include, but are not limited to, 1,3-butylene glycol, castor oil, corn oil, cottonseed oil, ethanol, fatty acid esters of sorbitan, germ oil, groundnut oil, glycerol, isopropanol, olive oil, polyethylene glycols, propylene glycol, sesame oil, water, mixtures thereof and the like. Excipients for preparation of compositions comprising a compound having formula (I) to be administered osmotically include, but are not limited to, chlorofluorohydrocarbons, ethanol, water, mixtures thereof and the like. Excipients for preparation of compositions comprising a compound having formula (I) to be administered parenterally include, but are not limited to, 1,3-butanediol, castor oil, corn oil, cottonseed oil, dextrose, germ oil, groundnut oil, liposomes, oleic acid, olive oil, peanut oil, Ringer's solution, safflower oil, sesame oil, soybean oil, U.S.P. or isotonic sodium chloride solution, water, mixtures thereof and the like. Excipients for preparation of compositions comprising a compound having formula (I) to be administered rectally or vaginally include, but are not limited to, cocoa butter, polyethylene glycol, wax, mixtures thereof and the like.

### EXAMPLE 1

### 1-(6-amino-3,5-difluoropyridin-2-yl)-8-chloro-6-fluoro-7-(3-hydroxyazetidin-1-yl)-4-oxo-1,4-dihydroquinoline-3-carboxylic acid

This compound was prepared as described in US 6,133,284, the specification of which is hereby incorporated by reference into this specification.

### EXAMPLE 2

### 1-deoxy-1-(methylammonium)-D-glucitol 1-(6-amino-3,5-difluoropyridin-2-yl)-8-chloro-6-fluoro-7-(3-hydroxyazetidin-1-yl)-4-oxo-1,4-dihydroquinoline-3-carboxylate

A mixture of EXAMPLE 1 (29.6Kg) and 1-deoxy-1-(methylamino)-D-glucitol (18.4Kg) was diluted with water (133Kg), stirred at 60°C until all solids dissolved, cooled to 38°C and held there until solid formed, cooled to 0°C and filtered. The filtrant was washed with isopropanol and dried at 50°C.

Ingredients were weighed out according to the formulas described in the tables of components and compositions of control tablet or capsule formulations shown in TABLE 1 TABLE 2 and TABLE 3. Polyvinylpyrrolidine (PVP) or hydroxypropylmethylcelluose (HPMC) in 15.5 mL (25% of total batch size: 50 g) of water, Mixtures of EXAMPLE 1 and Na₂CO₃ were dry mixed in a granulator for 5 minutes, treated with PVP or HPMC solution over 1 minute with water addition to assure optimal granulation, dried at 50°C until the loss on drying (LOD) was less than 1% (typically18 hours) and passed through a 20 mesh screen. The screened powder was blended with the extragranular for 3 minutes. The tablets were compressed to a hardness of 18-20 SCU using a Carver presser with tooling #A2201 Record compression forces or manually filled into appropriate size capsules to provide a fill of 500 mg/unit.

For the single pH dissolution medium experiment, 0.1 N HCl (pH 1.0) or 0.1 N HCl (900 mL) with various concentrations of HPMC (0.001%-1%) at 37°C ± 0.5°C were used with a USP Dissolution Apparatus 2 at a speed of 50 or 100 ± 4% rpm. One test tablet or capsule was added to the medium, and 10 mL of samples were removed at 15, 30, 45, 60, 120 and 240 minutes and assayed by UV absorption at 282 nm.

For the dual pH dissolution medium experiment, one test tablet or capsule was added to 0.1 N HCl (500 mL) at 37°C ± 0.5°C. Dissolution samples were removed at 15, 30, 45, 60, 120 and 240 minutes and assayed by UV absorption at 282 nm, and the dissolution medium was treated with 0.118M sodium phosphate buffer (pre-warmed to 37°C ± 0.5°C).

**TABLE 1**

| Ingredient | Formulation A | Formulation B | Formulation C | Formulation D |
|---|---|---|---|---|
| Intergranular | | | | |
| EXAMPLE 1 | 288.6 | 288.6 | 288.6 | 288.6 |
| HPMC | | | | 144.3 (powder) |
| PVP | 15.0 (powder) | 15.0 (soln) | 15.0 (soln) | |
| Na₂CO₃ | | | 75.0 | |
| Crospovidone | 25.0 | | | |
| Avicel | 144.0 | | | |
| water | q.s. | q.s. | q.s. | |

| Extragranular | | | | |
|---|---|---|---|---|
| Avicel | | 93.4 | 93.4 | |
| Crospovidone | 25.0 | 25.0 | 25.0 | |
| Mg Stearate | 2.5 | 2.5 | 2.5 | |
| | | | | |
| Tablet Wt. (mg) | 500 | 425 | 425 | 433.3 |

**TABLE 2**

| Ingredient | Formulation E (mg) | Formulation F (mg) | Formulation G (mg) | Formulation H (mg) |
|---|---|---|---|---|
| Intergranular | | | | |
| EXAMPLE 1 | 288.6 | 288.6 | 288.6 | 288.6 |
| HPMC E5 | 15.0 (soln.) | | 15.0 (soln.) | 15.0 (soln.) |
| PVP k30 | | 15.0 (soln.) | | |
| Na₂CO₃ | 75 | 75 | 75 | 75 |

| Extragranular | | | | |
|---|---|---|---|---|
| HPMC E5 | | 10 | 10 | 25 |
| Avicel PH 102 | 93.9 | 83.9 | 83.9 | 68.9 |
| Crospovidone | 25 | 25 | 5 | 25 |
| Mg Stearate | 2.5 | 2.5 | 2.5 | 2.5 |
| | | | | |
| Tablet Wt. (mg) | 500 | 500 | 500 | 500 |
| Hardness (SCU) | 18-20 | 18-20 | 18-20 | 18-20 |

**TABLE 3**

| Ingredient (mg) | Formulations | | |
|---|---|---|---|
| | Control | Polymer Solution | Polymer Powder |
| Intergranular | | | |
| ABT-492 (salt)* | 288.6 | 288.6 | 288.6 |
| Polymer | | 0.5-100 | 2.5-15 |
| water | q.s. | q.s. | q.s. |

| Extragranular | | | |
|---|---|---|---|
| Avicel | 1.83.9 | 83.9-183.4 | 168.9-181.4 |
| Crospovidone | 25 | 25 | 25 |
| Mg Stearate | 2.5 | 2.5 | 2.5 |
| Tablet wt. (mg) | 500 | 500 | 500 |

| | | | |
|---|---|---|---|
| *megluamine | | | |

Little or no drug was released in the low pH (1.2) medium within one hour for all tested formulations. Following adjustment to pH 7.5 for 2 hours, release of the drug from the control tablets were about 20% and almost the same as that from the formulation containing 0.1% polyvinyl pyrrolidinone (PVP K30). Dissolution of the drug was improved with an increase in amount of PVP K30. High dissolution extent (>80%) was obtained for formulations containing 1-3% PVP K30. Inclusion of hydroxypropylmethylcelluose (HPMC E5) showed a similar impact on drug dissolution.

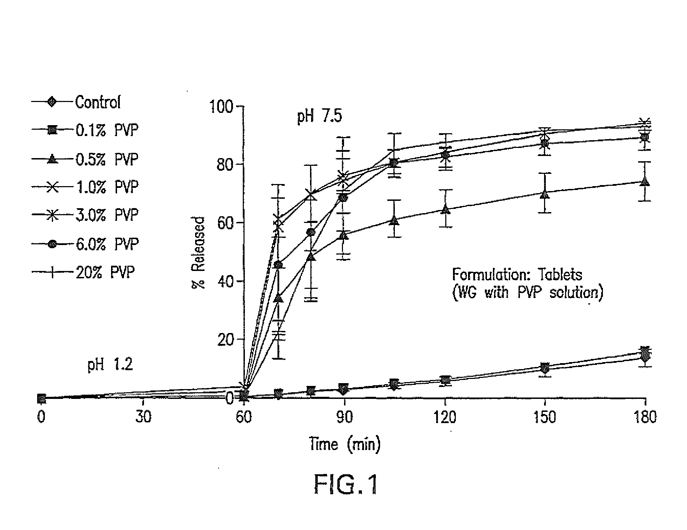

For the tablets made by adding PVP solution as granulation liquid with no Na₂CO₃, dissolution increases with moderate variability in spite of the release pH 1.0 medium being low. Average percentage released increases to 85% in higher pH medium at the 2 hour time point. Direct compression of drug with a higher percentage of HPMC (E5 LV) resulted in a reduction in dissolution variability although a large amount of HPMC would be expected to slow dissolution rate. These results are summarized in Figure 1.

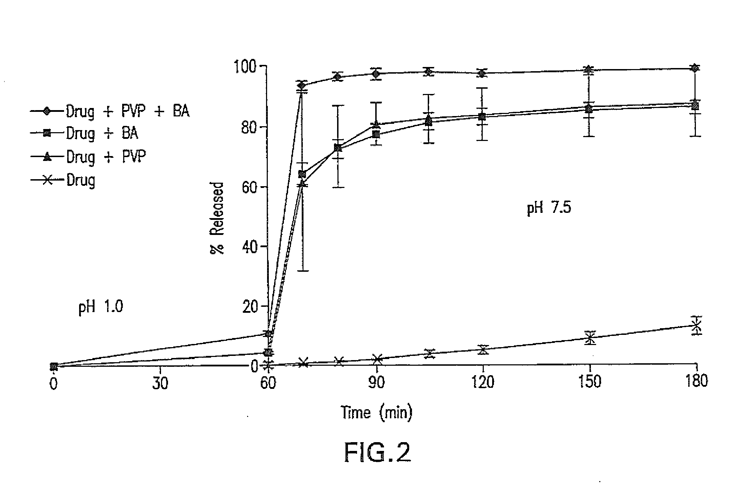

Dissolution of tablets granulated with PVP solution and Na₂CO₃ is rapid in the dual pH media. More interestingly, in pH 1.0 medium, there was about 12% of drug released at the 1 hour time point with an EXAMPLE 1 concentration about 20-fold greater than its intrinsic solubility. The pH of the solution at this time point was 1.0. The differences in dissolution rate/extant and variability among the formulations tested are summarized in TABLE 3 with data from the last pull point at pH 7.5 used as the example. These results are summarized in Figure 2.

**TABLE 4**

| | No Na₂CO₃ PVP Powder | No Na₂CO₃ PVP Solution | Na₂CO₃ PVP Solution |
|---|---|---|---|
| Percent EXAMPLE 1 Released at 2 hours | 78.28% | 88.37% | 99.77% |
| RSD* at 2 hours (percentage) | 55.7% | 12,2% | 0.51% |

| | | | |
|---|---|---|---|
| *relative standard deviation | | | |

These data show the increased dissolution of a drug in the presence of a pH modifier and polymer. As seen in Figure 2, there is little drug released into the low pH medium even after increasing the duration of the capsules in pH 1.0. However, duration time in low pH media does effect the dissolution profile of EXAMPLE 1 in the dual pH media after the pH has been adjusted to 7.5. In general, the shorter exposure time in low pH resulted in a higher extent dissolution.

Similar experiments were conducted with 5,5-diphenylhydantoin, sodium salt (NaPh). The solubility of 5,5-diphenylhydantoin at varying pH's is reported in J. Pharm. Sci. Vol. 82(3), March 1993. The dissolution study for the purpose of this invention was conducted in pH 7 buffer (900 mL) at 37°C ± 0.5°C with a paddle rotation speed of 50 rpm. Samples were removed at pre-determined time points and analyzed for concentration or drug. Formulations of NaPh are shown in TABLE 3.

**TABLE 5**

| Formulation: | E | F | G | H | I | J |
|---|---|---|---|---|---|---|
| Ingredients | | | | | | |
| NaPh | 3 g | 3 g | 3 g | 3 g | 3 g | 3 g |
| NaOH | - | 420 mg | 420 mg | 420 mg | | - |
| PVP | - | - | 1.2 g | 1.2 g | - | - |
| Lactose | - | - | | 3 g | 3 g | 3 g |
| Water (adj. to pH 7 with NaOH) q.s. | 3L | 3L | 3L | 3L | 3L | 3L |

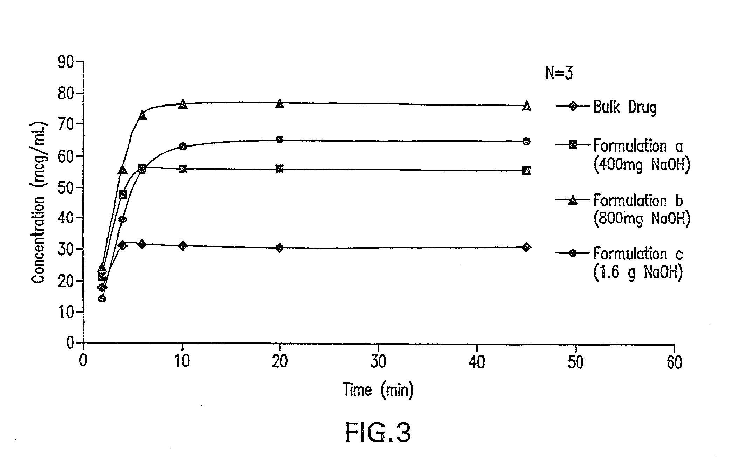

The results of the study, shown in Figure 3, illustrate the higher extent of dissolution. These data stand in contrast to the observation that 5,5-diphenylhydantoin, sodium salt forms an insoluble plug during dissolution at lower pH.

**TABLE 6**

| Ingredient (mg) | MEF | MEF-K | MEF-K+ BA | MEF-K + Polymer | MEF-K+ BA+ PVP | MEF-K+ BA+ HPMC |
|---|---|---|---|---|---|---|
| MEF | 100 | - | - | - | - | - |
| MEF-K | - | 115 | 117.3 | 115 | 117.3 | 117.3 |
| Na₂CO₃ (BA) | - | - | 29.2-48.9 | - | 29.2-48.9 | 29.2-48.9 |
| Polymer (In solution) | - | - | 4.8 | 4.8 | 3.1-12.2 | 6.1 |
| Tablet weight (mg) | 100 | 115 | 150-180 | 120 | 153-183 | 153-183 |

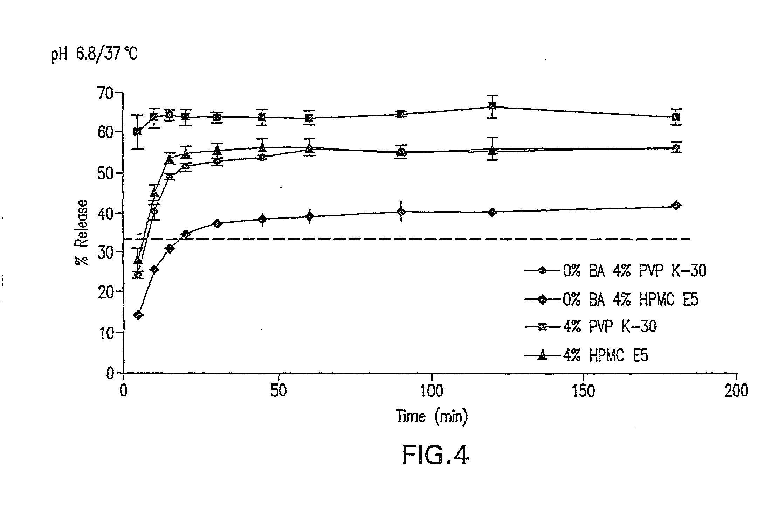

Similar experiments were conducted with potassium mefenamate (MBF-K). The intrinsic solubility (aqueous medium/lower pH) ofmefanamic acid is 0.0423 µg/mL. The dissolution study for the purpose of this invention was conducted in pH 6.8 buffer (900 mL) at 37°C ± 0.5°C with a paddle rotation speed of 50 rpm. Samples were removed at pre-determined time points and analyzed for concentration or drug. The results of this experiment are shown in Figure 4 and illustrate the improved dissolution of the formulation comprising potassium mefenamate. In this case, the concentration in the dissolution medium exceeded the solubility of the drug.

The foregoing is merely illustrative of the invention and is not intended to limit the same to the disclosed compounds and processes. Variations and changes which are obvious to one skilled in the art, as defined in the claims, are intended to be within the scope and nature of the invention.

## Claims

1. A composition comprising an ionizable drug or a salt thereof, a crystallization inhibitor and a pH modifier, said composition providing a measurable improvement in dissolution rate of the ionizable drug or a reduced variability in dissolution.

2. A composition according to claim 1 comprising a salt of an ionizable drug, a crystallization inhibitor and a pH modifier, said composition providing a measurable improvement in dissolution rate of the ionizable drug.

3. A composition according to claim 1 comprising 1-(6-amil-to-3,5-difluoropyridin-2-yl)-8-chloro-6-fluoro-7-(3-hydroxyazetidin-1-yl)-4-oxo-1,4-dihydroquinoline-3-carboxylic acid or a salt thereof, a crystallization inhibitor and a pH modifier, said composition providing a measurable improvement in dissolution rate of the ionizable drug.

4. A composition according to claim 1 comprising the megluamine salt of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid, a crystallization inhibitor and a pH modifier, said composition providing a measurable improvement in dissolution rate of the ionizable drug.

5. A composition comprising an ionizable drug or a salt thereof, a crystallization inhibitor and a pH modifier, said composition providing a measurable improvement in dissolution rate of the ionizable drug or a reduced variability in dissolution, for use in the treatment of disease in a patient.

6. A composition according to claim 5 comprising an ionizable drug, a crystallization inhibitor and a pH modifier, said composition providing a measurable improvement in dissolution rate of the ionizable drug, for use in the treatment of disease in a patient.

7. A composition according to claim 5 comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof, a crystallization inhibitor and a pH modifier, said composition providing a measurable improvement in dissolution rate of the ionizable drug, for use in the treatment of disease in a patient.

8. A composition according to claim 5 comprising the megluamine salt of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid, a crystallization inhibitor and a pH modifier, said composition providing a measurable improvement in dissolution rate of the ionizable drug, for use in the treatment of disease in a patient.
